# EUROPÄISCHE PATENTSCHRIFT

(11) **EP 3 339 517 B1**
(45) Veröffentlichungstag und Bekanntmachung des Hinweises auf die Patenterteilung: **21.05.2025**
(21) Anmeldenummer: 17210476.2
(22) Anmeldetag: 22.12.2017
(51) Int. Cl.: A61Q 19/10, A61K 8/02, A61K 8/34, A61K 8/44, A61K 8/46, A61K 8/60, E03C 1/04, E03C 1/046

(54) **VORRICHTUNG UND VERFAHREN ZUR ANREICHERUNG VON WASSER SOWIE ZUGEHÖRIGE FESTE, WASSERLÖSLICHE ZUSAMMENSETZUNG**
APPARATUS AND METHOD FOR THE ENRICHMENT OF WATER AND AN ASSOCIATED SOLID, WATER-SOLUBLE COMPOSITIONS
APPAREIL ET PROCÉDÉ POUR L'ENRICHISSEMENT DE L'EAU ET DES COMPOSITIONS SOLIDES, SOLUBLES DANS L'EAU, ASSOCIÉES

(30) Priorität: 22.12.2016 DE 102016015354; 22.12.2016 DE 202016008114 U
(43) Veröffentlichungstag der Anmeldung: 27.06.2018
(73) Patentinhaber: Showerplus Entwicklungs- und Vertriebsgesellschaft mbH, 31137 Hildesheim (DE)
(72) Erfinder: SACKEL, Rene, 31141 Hildesheim (DE)
(74) Vertreter: Eisenführ Speiser

(56) Entgegenhaltungen:
- WO-A1-2017/124154
- FR-A1- 2 331 521
- JP-A- 2000 079 357
- US-A- 3 207 445
- US-A- 3 402 892
- US-A- 3 777 982
- US-A1- 2009 121 042
- US-A1- 2012 255 623
- DATABASE GNPD [online] MINTEL; 15 December 2014 (2014-12-15), ANONYMOUS: "Scrub", XP055518746, retrieved from www.gnpd.com Database accession no. 2855915

## Beschreibung

### Kurzdarstellung der Erfindung

Hauterkrankungen sind divers und ihre Symptome reichen über u.a. Flecken, Schuppen, Risse, Ekzeme und/oder Rötungen. Viele Hauterkrankungen, wie beispielsweise Neurodermitis, beeinträchtigen die Betroffenen teilweise sehr stark. Die Behandlung und Pflege der betroffenen Haut benötigt hierbei eine spezielle Pflege, die neben der alltäglichen Körperhygiene zusätzlich auf die Haut aufgetragen werden muss. Diese zusätzliche Pflege ist zeitaufwendig und lästig. Kombinationsvorrichtungen, die die alltägliche Körperpflege mit der zusätzlichen Pflege verbinden können, sind daher nachgefragt.

Aber auch die schnelllebige und hektischere Zeit führt dazu, dass Kombinationsgeräte mit denen mehrere Sachen gleichzeitig erledigt werden können, nachgefragt sind. Hierbei steht insbesondere im Vordergrund das Leben und die alltäglichen Bedürfnisse in einer einfachen Form befriedigt und beschleunigt werden können. Insbesondere bei der täglichen Hygiene, wie beim Händewaschen, der Dusche oder dem Baden erfolgt meist eine Zeitersparnis, die gegebenenfalls zu einer verminderten Nutzungsmenge an Reinigungsmittel führt, was sich wiederum in einer mangelnden Hygiene wiederspiegeln kann.

Kombinationsgeräte zur Reinigung des Körpers sind bekannt, wie beispielsweise der DE 71 46 938 U1 zu entnehmen ist. Das Dokument betrifft eine Badebrause, in welche an einer bestimmten Position am Kopfstück Badezusätze eingeführt werden können.

In der DE 197 02 315 C1 ist ein druckloser Flüssigkeitsbehälter am Duschkopf zum Beimischen von Zusatzstoffen angebracht, wobei der Austritt der Flüssigkeit aus dem Behälter durch die Schwerkraft bemessen ist.

Zum weiteren Stand der Technik wird hingewiesen auf WO 2017/124154, welches ein Haarbehandlungssystem beschreibt, insbesondere eine Tablette, die in ein Duschkopf einsetzbar ist.

Dann wird hingewiesen auf US 2012/0255623, welches einen Formkörper zum Einsatz in Wasserhähnen beschreibt, der Formkörper durch Gehalte an Natriumdodecylsulfat und/ oder Natriumcylensulfat bemisst.

Ferner wird als weiterer Stand der Technik hingewiesen auf FR 2331521 A1, welches die Verwendung von Tensiden fester Konsistenz offenbart.

In den Gebrauchsmustern DE 20 2008 013 784 U1 als auch DE 20 2006 017 888 U1 werden Duschköpfe mit Griffstück offenbart, wobei im Griff sich ein Reservoir für eine entsprechende Füllung befindet.

Nachteil dieser Geräte ist, dass nur versetztes Wasser mit entsprechendem Material verwendet werden kann. Nur durch die Entfernung des Materials aus den Duschköpfen oder Griffen lässt sich wieder reines Wasser verwenden.

Ein weiterer Nachteil ist, dass in diese Vorrichtungen, eine lose Zusammenstellung von Wirkstoffen, wie beispielsweise Mineralsteinen verwendet wird oder eine Badetablette eingesetzt wird, die üblicherweise für Bäder vorgesehen ist und zumeist Salze enthält.

Die Aufgabe der vorliegenden Erfindung ist es, eine Zusammensetzung zur Verfügung zu stellen, die die angegebenen Nachteile überwindet.

Die Aufgabe wird erfindungsgemäß mit einer Zusammensetzung mit den Merkmalen nach Anspruch 1 gelöst. Vorteilhafte Weiterbildungen sind in den Unteransprüchen beschrieben, wie auch ein Verfahren zur Anreicherung von Wasser mit einer Zusammensetzung nach einem der Ansprüche 1 bis 7.

Die Zusammensetzung kann in einer Vorrichtung zur Anreicherung von Wasser eingesetzt werden, wobei diese mindestens zwei getrennte Kammern umfasst.

Die mindestens eine Kammer umfasst in einer Ausführungsform der vorliegenden Erfindung einen Aufnahmebehälter, der Elemente zur Aufnahme einer Zusammensetzung enthält. Die Elemente halten die Zusammensetzung mittig in dem Aufnahmebehälter. Alternativ oder zusätzlich kann vorgesehen sein, dass der Aufnahmebehälter für die Zusammensetzung im Wassereingangsbereich und Wasserausgangsbereich Filter aufweist, die die Zusammensetzung in dem Aufnahmebehälter halten.

Das Verteilerelement ist in einer Ausführungsform, ein Bypass-Modul oder mindestens ein Element, das den Wasserfluss von einem in einen anderen Aufnahmebehälter steuern kann. In einer bevorzugten Ausführungsform hat das Verteilerelement mindestens drei Einstellmöglichkeiten.

In einer Ausführungsform umfasst die Vorrichtung ein Einfüllsystem, zum Auswechseln der Zusammensetzung und/oder Befüllen des Aufnahmebehälters mit der Zusammensetzung umfasst.

Die Ausführungsform umfasst eine Mischkammer zur Verwirbelung des fließfähigen Materials. In einer Ausführungsform bevorzugten Ausführungsform erfolgt die Verwirbelung mittels Sauerstoffdüse, besonders bevorzugt mittels Venturi-Düse.

Die erfindungsgemäße Zusammensetzung dient insbesondere der Behandlung der Haut und/oder der Haare.

Seifen kommen zumeist in Stückform vor und werden zur Reinigung und Pflege der Haut verwendet. Diese weisen jedoch eine begrenzte Auswahl an Wirkstoffen auf. Weiterhin ist eine homogene ausreichende Auflösung der Seifen im Wasserfluss nicht gegeben, da diese nicht ausreichend in diesem alleinig aufgelöst werden, um eine Behandlung des Körpers und/oder der Haare zu ermöglichen. Üblicherweise muss das Seifenstück zwischen den Händen hin- und herbewegt werden, um eine Aufschäumung zu generieren.

Weiterhin sind Flüssigseifen, Lotionen und Desinfektionsmittel bekannt, die in flüssiger Form auf die Haut aufgebracht werden, bzw. mit Wasser vermischt werden, beispielsweise in Form von Badezusätzen. Der Nachteil der flüssigen Komponenten ist, dass diese nicht homogen mit dem Wasser vermischt werden können, da deren Zugabe von der Beigabegeschwindigkeit abhängt.

Die Aufgabe der vorliegenden Erfindung ist eine Zusammensetzung zur Verfügung zu stellen, die sich innerhalb eines Wasserstroms homogen auflöst ohne Konzentrationsspitzen zu erreichen. Die Zusammensetzung soll weiterhin für die Verwendung in einer Dusch-, Badevorrichtung, Armatur oder einem Applikator geeignet sein und vorzugsweise innerhalb eines Dusch-Zyklus lösbar sein. Ein weiterer Vorteil der vorliegenden Zusammensetzung ist, dass die Zusammensetzung keine Umverpackung benötigt, wodurch kein unnötiger Abfall generiert wird.

Die vorliegende Erfindung umfasst daher auch eine feste, wasserlösliche Zusammensetzung zur Behandlung der Haut und/oder der Haare, wobei die Fettphase eine Kombination aus 2-[3-(dodecanoylamino)propyl- dimethylazaniumyl]acetat (Cocamidopropyl Betaine, CAPB), (2R,3R,4S,5S,6R)-2-Decoxy-6-(hydroxymethyl)tetrahydropyran-3,4,5-triol (Decyl-Glucosid), 1,2- Propandiol (Propylenglycol), Kokosglucoside (Cocoglucoside), Natriumcocoylisethionat.

Die Zusammensetzung weist in einer bevorzugten Ausführungsform die Form eines dreidimensionalen Sticks auf, der besonders bevorzugt die Grundfläche eines gleichschenkligen Kreuzes aufweist.

In einer Ausführungsform der vorliegenden Erfindung enthält die Zusammensetzung Mittel zur Desinfizierung, medizinischen Behandlung, Pflege der Haut und/oder der Haare und/oder eine Parfüm- und/oder Farbphase.

Die vorliegende Erfindung betrifft weiterhin ein Verfahren zur Anreicherung von Wasser, wobei die oben beschriebene Vorrichtung und eine entsprechende Zusammensetzung verwendet wird.

Die Aufgabe wird weiterhin durch die Ausführungsformen in den Ansprüchen gekennzeichnet und durch die Ausführungen in der Beschreibung, den Beispielen und den Zeichnungen näher beschrieben.

### Abbildungen

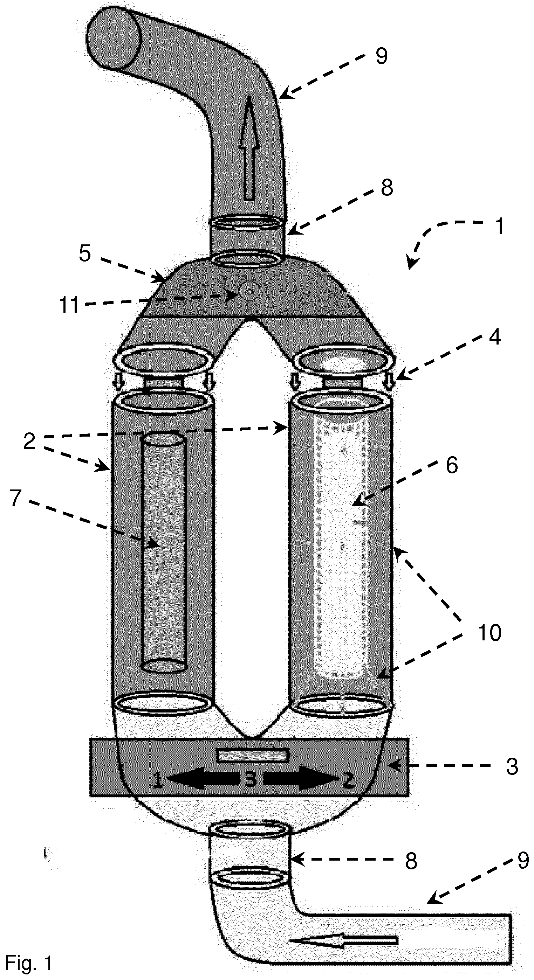

- Fig. 1: Schematische Darstellung der Vorrichtung mit zwei Anreicherungskammern und einem Aufnahmebehälter mit Duschkopf.

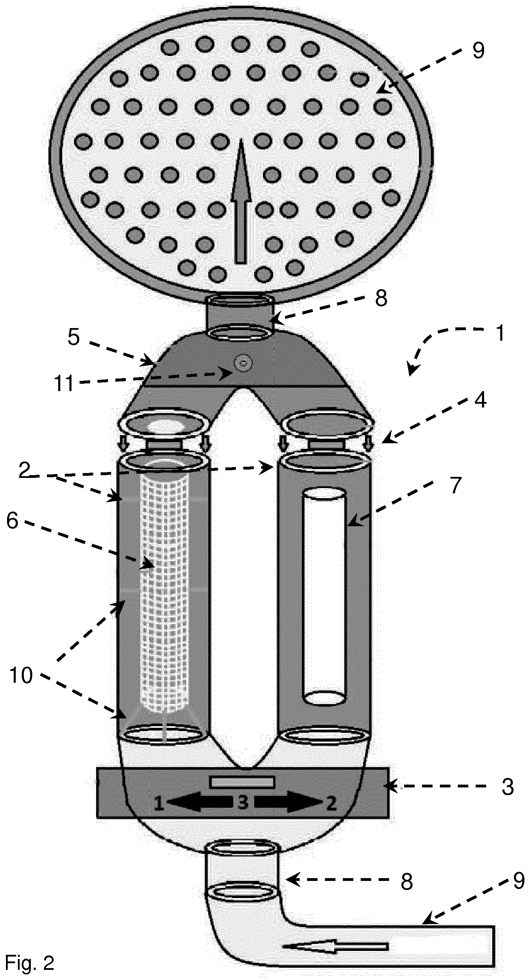

- Fig. 2: Schematische Darstellung der Vorrichtung mit zwei Anreicherungskammern, einem Aufnahmebehälter und einfachem Wasseranschluss.

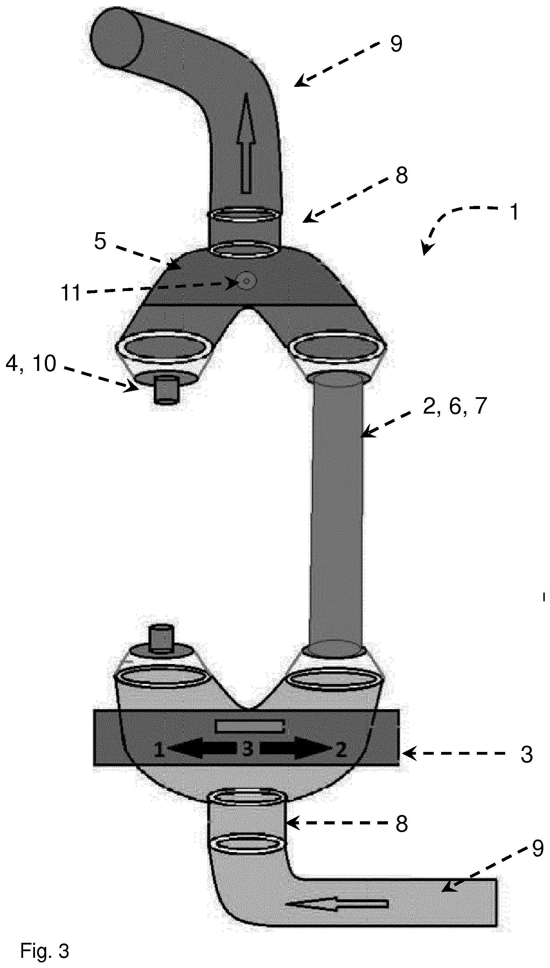

- Fig. 3: Schematische Darstellung der Vorrichtung mit zwei Anreicherungskammern in Form eines Aufnahmebehälter und einfachem Wasseranschluss.

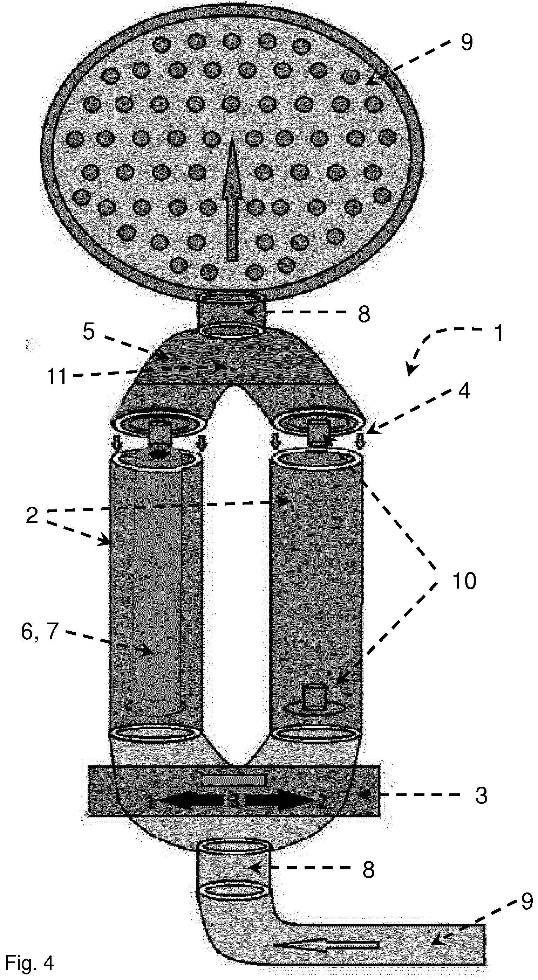

- Fig. 4: Schematische Darstellung der Vorrichtung mit zwei Anreicherungskammern in Form eines Aufnahmebehälter mit Duschkopf.
- Fig. 5: Schematische Darstellung einer möglichen Befestigung des Aufnahmebehälters.
- Fig. 6: Darstellung einer erfindungsgemäßen Zusammensetzung in Form eines dreidimensionalen mit der Grundfläche eines gleichschenkligen Kreuzes - Perspektivische Ansicht.
- Fig. 7: Schematische Darstellung einer möglichen Ausführungsform der vorliegenden Vorrichtung. a)-d) zeigen die Vorrichtung in ihrer äußeren Ausführung; e)-h) zeigen den Innenaufbau der Vorrichtung; i) zeigen eine vereinfachte Schematische Darstellung der Vorrichtung mit der Mischkammer und dem Wasserdurchlauf, sowie dem Verteiler. Seitliche Darstellung der Vorrichtung a) und e) sowie b) und f) beider Seiten; c) und g) perspektivische Darstellung der Vorrichtung; d) und h) Vorderansicht.
- Fig. 8: Schematische Darstellung einer möglichen Verwendung der vorliegenden Vorrichtung. A) perspektivische Darstellung der Vorrichtung an einer handelsüblichen Duschvorrichtung; b) seitliche Darstellung der Vorrichtung an einer handelsüblichen Duschvorrichtung, wobei diese an der Mischbatterie angebracht ist.
- Fig. 9: Schematische Darstellung einer möglichen Ausführungsform der vorliegenden Vorrichtung, bei der diese direkt am Duschkopf angebracht ist.

### Detaillierte Darstellung der Erfindung

Die heutige Zeit ist schnelllebig, wodurch Vorrichtungen, die einem den Alltag erleichtern und/oder alltägliche Prozesse beschleunigen gefragt sind.

Die vorliegende Erfindung betrifft daher eine Vorrichtung zur Anreicherung von Wasser. Neben der Anreicherung von Wasser mit verschiedenen Reinigungsmitteln und Seifen, die zur Säuberung der Haut und/oder Haaren geeignet sind, können auch Pflegeprodukte beigemischt werden, die zur Pflege von Haut und/oder Haaren geeignet sind. Viele Erkrankungen der Haut, wie beispielsweise Neurodermitis, benötigen spezielle Pflege, ohne welche die Betroffenen durch die Veränderungen der Hautstruktur u.a. an Hautrötungen, Risse und/oder Juckreiz leiden. Um die Beeinträchtigung der Betroffenen, die unter Hautkrankheiten leiden, können spezielle Mittel dem Wasser beigemischt werden. Diese Produkte können Basispflegeprodukte umfassen aber auch spezielle Wirkstoffe wie Vitamine und/oder Mineralien und/oder antiseptische, antimykotische und/oder entzündungshemmende Wirkstoffe enthalten. Die Beimischung der verschiedenen Produkte und Wirkstoffe kann je nach Anwender angepasst werden und erspart dem Anwender zusätzliche Zeit zur Pflege nach dem Duschvorgang. Daneben können neben Mitteln zur Reinigung, Pflege und/oder Behandlung auch Medien Verwendung finden die für das "Spaßduschen" geeignet sind. Insbesondere für Kinder können Medien verwendet werden, die das Wasser färben und/ oder Glitzereffekte einbringen.

Die Vorrichtung zur Anreicherung von Wasser 1 kann in einer Ausführungsform separat ausgebildet sein, indem sie zwischen handelsübliche Armaturen, Duschvorrichtungen (z. B. Duschkopf), Schlauchverbindungen und den Wasseranschluss eingebracht wird. Die Vorrichtung zur Anreicherung von Wasser 1 kann neben einer kompletten Duschvorrichtung auch ein Duschkopf, ein Applikator oder eine Armatur sein. Im Duschkopf kann die Vorrichtung beispielsweise im Griff angeordnet sein. In einer bevorzugten Ausführungsform handelt es sich bei der Vorrichtung, um eine zwischenschaltbare Vorrichtung, die zwischen handelsübliche Armaturen und Duschvorrichtungen eingebracht werden kann. In einer Ausführungsform wird ein Duschkopf verwendet, der aus korrosionsarmen Materialien hergestellt wurde. Aufgrund pflegender Medien und Medien zur Behandlung von Hautproblemen und/oder Erkrankungen kann in den herkömmlichen Duschvorrichtungen, wie Duschköpfen, eine vermehrte Korrosion auftreten, die im Zusammenhang mit den Bestandteilen dieser Produkte, meist Salze und/oder andere Ionen steht. Die Duschvorrichtungen, wie Duschköpfe, können jegliche Variation aufweisen, wie beispielsweise mit/ohne Massagefunktion, Einstellung der Wasserhärte, Regenstrahl, und andere Funktionen beinhalten die im Stand der Technik bekannt sind. Alternativ kann die Vorrichtung 1 Teil einer Armatur oder einer Duschvorrichtung (z. B. eines Duschkopfes) sein. Der Duschkopf kann als Teil der Vorrichtung verschiedene Möglichkeiten der Wasserherausgabe beinhalten, wie beispielsweise mit/ohne Massagefunktion, Einstellung der Wasserhärte, Regenstrahl, und andere Funktionen. In einer bevorzugten Ausführungsform weist der Duschkopf korrosionsarme Materialien auf. Diese sind insbesondere für die Anwendung mit Salzen, wie Mineralsalzen, vorteilhaft, da Salze die Korrosion beschleunigen.

Die Vorrichtung 1 umfasst dabei einen Verteiler 3, mindestens zwei getrennte Kammern 2, ein Einfüllsystem 4 und eine Mischkammer 5.

Der Verteiler 3 der vorliegenden Vorrichtung ermöglicht das Umschalten des Wasserflusses aus der Wasserleitung, d. h. der Wassereintrittsöffnung auf eine oder beide der mindestens zwei Kammern 2. In einer Ausführungsform ist der Verteiler ein Bypass-Modul 3. Das Bypass-Modul 3 umfasst bei einer Ausführung mit zwei Kammern 2 drei Einstellmöglichkeiten. Diese Einstellmöglichkeiten erlauben es den Wasserfluss so zu regeln, dass dieser entweder durch eine der beiden Kammern 2 oder durch beide Kammern 2 fließt. Sind mehr als zwei Kammern 2 vorhanden, kann der Verteiler 3 auch mehrere Einstellmöglichkeiten haben, sodass der Wasserfluss jedes Medium 7 einzeln oder alle gemeinsam erreicht. Die Einstellmöglichkeiten am Verteiler 3 können durch Elemente wie Regler ausgewählt werden. In einer bevorzugten Ausführungsform sind die Regler Hebel, Hähne, Schieber oder Knöpfe. In einer besonders bevorzugten Ausführungsform sind die Regler Schieber. Der Verteiler 3 ist durch einen Standardanschluss (DIN-Anschluss) 8 mit der Wasserzuleitung 9 verbunden.

Die Vorrichtung kann an einer oder beiden Seiten des Wassereingangs bzw. Wasserausgangs durch Standardverbindungen mit der Wasserzuleitung bzw. der Wasserweiterleitung, wie beispielsweise dem Wasserschlauch, Duschkopf, Mischbatterie und/oder Armatur verbunden werden. Die Verbindungen sind zumeist standardisierte DIN-Anschlüsse. In einer bevorzugten Ausführungsform handelt es sich bei der Verbindung um einen Drehanschluss. Dieser hat den Vorteil, dass sich beispielsweise der Duschschlauch nicht verdreht, sondern sich die Vorrichtung und/oder der Schlauch mitdreht.

In einer Ausführungsform enthält der Verteiler 3 ein Rückschlagventil. Das Rückschlagventil soll eine Rückleitung des Wassers in die Zuleitung 9 verhindern. Das Rückschlagventil kann aber auch in einer der Anreicherungskammern 2 und/oder der Misch-/Verwirbelungskammer 5 enthalten sein, um einen Rückfluss des ggf. bereits angereicherten Wassers zu verhindern.

Die mindestens zwei Kammern 2 der vorliegenden Erfindung dienen der Aufnahme des Mittels zur Reinigung, Pflege und/oder Behandlung der Haut und/oder der Haare oder des Durchflusses des klaren nicht angereicherten Wassers. In einer Ausführungsform sind die Kammern 2 so ausgerichtet, dass sie einen Aufnahmebehälter 6 aufnehmen können oder enthalten. Der Ausnahmebehälter 6 kann verschiedene Ausführungen aufweisen, wie beispielsweise ein mindestens ein Gitter oder Sieb, welches mittig in mindestens einer Anreicherungskammer 2 angeordnet ist, ein Gitter oder Sieb, das im Bereich der Wassereinlauföffnungen in die mindestens eine Kammer 2 angeordnet ist. Die Aufgabe des Aufnahmebehälters ist, dass mögliche größere Teile des Mediums 7 zurückgehalten werden und ein verstopfen der Leitungen oder Vorrichtungen verhindern. Um den Kontakt von Wasser und Medium 7 zu gewährleisten, umfasst der Aufnahmebehälter 6 in einer Ausführungsform mindestens zwei Öffnungen auf den sich gegenüberliegenden Seiten in der Fließrichtung des Wassers. In einer bevorzugten Ausführungsform ist der Aufnahmebehälter 6 permeabel. Die Permeabilität kann durch ein feinporiges Material, ein Sieb, Gitter oder Ähnliches erreicht werden. Vorzugsweise besteht der Aufnahmebehälter 6 aus feinporigen Materialien und/oder Elementen, wodurch grobes Medium zur Reinigung, Pflege und/oder Behandlung in dem Behälter zurückbleibt und ein Verstopfen der Leitungen verhindert. Der Aufnahmebehälter 6 dient daher als Rückhaltevorrichtung für Material 7, um ein Verstopfen der Wasserleitungen bzw. folgender Düsen zu verhindern.

Das Medium 7 zur Reinigung, Pflege und/oder Behandlung der Haut und/oder der Haare wird in die mindestens eine Kammer 2, vorzugsweise in den Aufnahmebehälter 6 platziert und durch den Wasserfluss in der Anreicherungskammer 2 teilweise in Wasser aufgenommen. Um eine optimale Anreicherung des Wassers mit dem Medium 7 zu erreichen, ist der Aufnahmebehälter 2 bevorzugt mittig in der Anreicherungskammer 2 angeordnet. Die Befestigung des Behälters erfolgt hierbei durch Verbindungselemente 10, die dem Wasserdruck in der Kammer standhalten. Diese Elemente 10 können fest mit der Anreicherungskammer 2 und/oder dem Aufnahmebehälter 6 verbunden sein. In einer Ausführungsform ist der Aufnahmebehälter 6 aus der Anreicherungskammer 2 entfernbar angeordnet, sodass eine Reinigung des Behälters von Rückständen des Mediums 7 erleichtert wird. Die Verbindungselemente 10 können Streben, Halterungen, Schrauben, Bolzen, Nieten, Schweißverbindungen und/oder Bajonettverschlüsse sein. Die Verbindungselemente 10 sind jedoch nicht auf die vorherige Ausführung beschränkt, sondern können jedes in der Befestigungstechnik bekannte Element sein.

In einer alternativen Ausführungsform können die mindestens zwei Kammern 2, die zur Anreicherung des Wasser dienen können, selbst die Aufnahmebehälter 6 sein. Hierbei wird in die Anreicherungskammer 2 eine Kartusche oder eine Zusammensetzung in der sich das Medium 7 zur Reinigung, Pflege und/oder Behandlung befindet, eingebracht. Diese Kartusche wird in einer Ausführungsform an das Verteilerelement 3, auf der einen Seite und die Misch- und/oder Verwirbelungskammer 5, auf der anderen Seite, befestigt. Die Befestigung entspricht hierbei dem Einfüllsystem 4 und kann durch verschiedene Verbindungselemente 10 erfolgen, die eine reversible Befestigung der Kartusche ermöglichen. Möglichkeiten reversibler Verbindungen im Stand der Technik allgemein bekannt und umfassen, sind jedoch nicht beschränkt auf Ratshalteelemente, Klippelemente und/oder Schnappelemente. In einer bevorzugten Ausführungsform umfasst das Einfüllsystem 4 jeweils für einen Aufnahmebehälter 2 zwei gefederte Vorsprünge, wobei der eine Vorsprung sich auf Seiten der Mischvorrichtung und der andere sich auf Seiten des Zuflusses befindet. In einer Ausführungsform weist die mindestens eine Kammer 2 eine komplementäre Öffnung auf, die der Form der Kartusche und/oder Zusammensetzung entspricht. Das Einfügen des Mediums 7 in Form der Zusammensetzung und/oder der Kartusche in die Öffnung, dient der besseren Positionierung und Einführung des Mediums 7 in die mindestens eine Anreicherungskammer 2. Ein Vorteil der Öffnung mit komplementärer Form zur Zusammensetzung oder Kartusche ist, dass beispielsweise nur in einer Kammer 2 sich Medium befindet, wobei die zweite Kammer frei von Zusätzen bleiben kann. In einer bevorzugten Ausführungsform handelt es sich bei der Form der Öffnung um ein Kreuz, wie beispielsweise dem Beispiel 1 zu entnehmen ist.

Das Einfüllsystem 4 der erfindungsgemäßen Vorrichtung 1 ermöglicht das Auswechseln und/oder Befüllen des Aufnahmebehälters 6. Das Einfüllsystem 4 kann jegliche im Stand der Technik mögliche Ausführungsform aufweisen, die es ermöglicht den Aufnahmebehälter 6 zu befüllen. Das Einfüllsystem 4 kann demnach eine Schraubverbindung, Scharnierverbindung, Klippverbindung, Schnappverschlüsse, Spannverschlüsse, Bajonettverschlüsse, Riegelverschlüsse oder eine Schiebeverbindung sein. In einer Ausführungsform umfasst das Einfüllsystem 4 je Anreicherungskammer 2 mindestens eine Scharniervorrichtung, Klippvorrichtung oder einen Bajonettverschluss auf. Die Verbindung ermöglicht es die Anreicherungskammer 2 von der folgenden Misch- und/oder Verwirbelungskammer 5 zu trennen. Diese Trennung kann komplett oder teilweise sein. Bei einer kompletten Abtrennung wird die Misch- und/oder Verwirbelungskammer 5 mit den an ihr befestigten Elementen von der Anreicherungskammer 2 und den daran befestigten Anschlüsse und Elemente gelöst. infolge dessen lässt sich der Aufnahmebehälter 6 befüllen. Bei einer teilweisen Abtrennung bleiben die Teile in einem Bereich miteinander verbunden, sodass ein Umklappen möglich ist. Die Einfüllvorrichtung 4 ist bevorzugt zwischen den mindestens zwei Anreicherungskammern 2 und der Misch- und/oder Verwirbelungskammer 5 angeordnet. Sie kann aber auch zwischen dem Verteiler 3 und der Anreicherungskammer 2 angebracht sein.

In einer Ausführungsform weist mindestens eine Anreicherungskammer 2 ein System zur Bestimmung der Füllmenge an Medium 7 auf. Das System kann die Anreicherungskammer 2 selbst sein, indem diese transparent oder teilweise transparent gestaltet ist und die Füllmenge optisch bestimmt werden kann. Das System kann aber auch eine mechanische oder elektrische Flüssigkeitsanzeige sein.

Die Misch- und/oder Verwirbelungskammer 5 der erfindungsgemäßen Vorrichtung dient der Verwirbelung des fließfähigen Materials. Das Medium 7 und/oder Wasser kann aus mindestens einer Kammer oder mehrehren Kammern vermischt werden. In einer Ausführungsform erfolgt die Vermischung aus den mindestens zwei Anreicherungskammern 2. Das Wasser wird hier mit Medium zum Reinigen, Pflegen und/oder zur Behandlung aus den jeweiligen Anreicherungskammern 2 vermischt. Die Vermischung aus den vorzugsweise zwei Kammern 2, kann auch die Konzentration des Mediums 7, das abgegeben wird beeinflussen, indem beispielsweise nur in einer Kammer 2 Medium 7 enthalten ist und klares Wasser aus der zweiten Kammer 2 in der Mischkammer mit dem angereicherten Wasser aus der ersten Kammer 2 vermischt wird. Die Konzentration an Medium und/oder das Mischungsverhältnis kann benutzerdefiniert bestimmt und verwendet werden. In einer Ausführungsform erfolgt die Vermischung durch eine Verwirbelung mittels einer Sauerstoffdüse 11. Die regelbare Sauerstoffzufuhr in die Misch- und/oder Verwirbelungskammer ermöglicht nicht nur eine optimale Vermischung des Mediums mit Wasser, sondern ermöglicht auch die Schaumproduktion bei schäumenden Medien und/oder die Wasserstrahlhärte zu steuern. Die Zugabe von Luft führt auch zu einer Verringerung des Wasserverbrauchs. In einer bevorzugten Ausführungsform ist die Sauerstoffdüse 11 eine Luft-Injektor Düse, die eine zuschaltbare, regulierbare Eingangsöffnung/Austrittsöffnung für Luft enthält. In einer besonders bevorzugten Ausführungsform ist die Düse einer Venturi-Düse.

In einer Ausführungsform kann die benutzerdefinierten Verwendung zusätzlich durch elektrische Elemente vereinfacht werden. Elektrische Elemente umfassen beispielsweise automatisierte Schalter oder Verteilerelemente die mittels elektronischer Kommunikation vorzugsweise per Fernsteuerung gesteuert werden können. Möglichkeiten der elektronischen Kommunikation umfassen, sind jedoch nicht beschränkt auf, Bluetooth, Funknetze, wie WLAN, Infrarot, ZigBee, NFC, Radiofrequenzen, IrDA und/oder optischer Richtfunk (FSO). Mithilfe dieser Technologien können die Benutzereinstellungen vor dem Duschen und Baden nicht nur vor einem solchen eingestellt werden, Benutzervorlieben können auch abgespeichert werden. Neben den Benutzervorlieben können mithilfe dieser Technologie auch der Verbrauch an Medium 7 an eine Zentraleinheit übermittelt werden, wodurch beispielsweise Nachschub an Medium 7 automatisiert nachbestellt werden könnte.

An die Misch- und/oder Verwirbelungskammer 5 kann mittels eines geläufigen DIN- Anschlusses 8 eine Armatur, ein Wasserhahn, eine Duschvorrichtung, insbesondere Duschkopf, eine flexiblen Leitung, wie ein Duschschlauch oder andere Wasserauslassvorrichtungen 9 angebracht werden.

Alle Verbindungsstücke und Elemente sind in einer Ausführungsform durch herkömmliche Dichtungselemente abgedichtet, sodass Wasser nicht aus der Vorrichtung austreten kann. Um zu verhindern, dass Wasser während des Einfüllvorgangs des Mediums austritt und spritzt, ist in einer Ausführungsform ein Wasserstop eingebaut, der den Wasserfluss im oder vor dem Verteiler zurückhält, wenn das Einfüllsystem in Benutzung ist.

In einer weiteren Ausführungsform der vorliegenden Erfindung ist bei der Wassereinlassöffnung der Vorrichtung 1, in welcher das Wasser in die Vorrichtung 1 fließt, ein Wasserrad, eine Turbine oder jeglicher dem Fachmann bekannte wasserbetriebene Energieerzeuger angeordnet. Der wasserbetriebene Energieerzeuger hat die Aufgabe durch den Wasserfluss in die Vorrichtung 1 Strom zu generieren, mit welchem die Vorrichtung die elektrischen Elemente zusätzlich und/oder alternativ speisen kann. Die erzeugte Energie kann jedoch auch dafür verwendet werden, um die Vorrichtung 1, die beispielsweise die Form eines Applikators, einer Mischbatterie und/oder einer Duschvorrichtung aufweist, zu beleuchten und/oder nur bestimmte Elemente wie die Produktbezeichnung oder das Verteilelement zu beleuchten.

Das Medium 7 zur Reinigung, Pflege und/oder zur Behandlung von Haut und/oder Haaren kann je nach Anreicherungskammer 2 bzw. Aufnahmebehälter 6 verschiedene Gestaltungen umfassten. Die Gestaltung des Mediums kann eine Flüssigkeit, ein Gel, oder eine feste Form sein. In einer Ausführungsform ist das Medium 7 ein Granulat oder eine Kartusche, Patrone oder Stick. Das Medium 7 kann hierbei für eine Anwendung ausgerichtet sein, indem das Granulat oder der Stick eine Auflösezeit einer durchschnittlichen Dusche hat. In einer Ausführungsform löst sich das Granulat oder der Stick zur einmaligen Verwendung innerhalb von 2 min bis 45 min. In einer bevorzugten Ausführungsform ist die Zeit der Auflösung zwischen 5 min und 30 min. In einer besonders bevorzugten Ausführungsform beträgt die Auflösezeit 6 min bis 15 min.

Das Medium 7 weist in einer Ausführungsform Sollbruchstellen auf, die eine komplette Auflösung dessen ermöglichen. Die Zusammensetzung des Mediums 7 besteht aus Reinigungsmedien, Pflegemedien und/oder Mitteln zur Behandlung von Haut und/oder Haaren.

In einer Ausführungsform umfasst das Medium 7 Bestandteile zur Reinigung, Pflege und/oder Behandlung der Haut, die in eine Form gepresst wurden. Diese gepresste Form kann jegliche bekannte Form sein. Bevorzugt besteht die Form aus einem länglichen Stift. Dieser Stift oder Stick löst sich bei Kontakt mit Wasser Schritt für Schritt auf. Die Auflösung erfolgt auch unter Nutzung der Sollbruchstellen in der gepressten Form. Das gepresste Medium 7 kann auch aus einer bestimmten chemischen Zusammensatzung bestehen, die einzelne Pflegestoffe, Reinigungsstoffe und/oder Stoffe zu Behandlung von Haut und/oder Haaren umfasst.

In einer Ausführungsform wird das Medium 7, wie z.B. Duschgel, erhärtet, sodass es eine gummiartige bis starre Struktur aufweist. Diese Struktur kann jegliche Form sein. Bevorzugt ist die Form der gummiartigen bis starren Struktur ein Stick. Dieser löst sich wie bereits der gepresste Stick in Verbindung mit Wasser nach und nach auf.

In einer Ausführungsform kann das Medium 7 zur Reinigung, Pflege und/oder Behandlung der Haut und/oder Haare die Form eines Fluid aufweisen. Dieses kann in einer Kartusche, Patrone oder anderen Behältnissen eingefüllt oder in diesen bereits nicht nachfüllbar vorhanden sein. Die Kartusche, Patrone oder anderes Behältnis 6 umfasst dabei Löcher durch die das Liquid fließen kann. Zur Regulierung der Menge und/oder Verwendungsdauer des Fluids bei der Reinigung, Pflege und/oder Behandlung können die Löcher unterschiedliche Größen aufweisen, die Menge an Löchern variieren und/oder die Viskosität des Fluids unterschiedlich sein. Je dickflüssiger (weniger fließfähig) das Fluid ist, desto länger ist die Verwendungsdauer und/oder geringer die Menge die eingesetzt werden muss. Je niedriger die Viskosität des Fluids, desto dünnflüssiger (fließfähiger) ist dieses und die Verwendungsdauer des Fluids ist kürzer. In letzterem Fall ist die Menge an verwendetem Fluid auch höher.

Neben den bereits vorherig bezeichneten Mitteln zur Reinigung, Pflege und/oder Behandlung kann das Medium nur oder zusätzlich Mitteln enthalten, die das Wasser anfärben, Glitzer enthalten und für ein "Spaßduschen" geeignet sind.

Die vorliegende Erfindung betrifft daher auch ein Medium 7, das als feste, wasserlösliche Zusammensetzung innerhalb eines Wasserstroms, beispielsweise einer Duschvorrichtung, einem Applikator mit Wasserdurchfluss, Armarturen oder anderen Vorrichtungen zur Abgabe von Wasser, eingesetzt werden kann und die Bestandteile an das Wasser abgibt. Die Zusammensetzung ist vorzugsweise derart ausgebildet, dass diese in eine zuvor beschriebene Vorrichtung eingebracht werden kann. Die Zusammensetzung wird derart in den Wasserstrom eingebracht, dass während des Wasserflusses Wasser die Zusammensetzung umschwemmt, wodurch sich dieser schrittweise auflöst und die Wirkbestandteile vorzugsweise homogen an den Wasserstrom abgegeben werden. Es erfolgt daher eine automatische und hinhaltende Freisetzung der Wirkbestandteile.

Fest bezeichnet hierbei die anfängliche Konsistenz der Zusammensetzung, ohne dass diese Wasser ausgesetzt wurde. Die feste Materie der Zusammensetzung erlaubt es die Zusammensetzung zu fassen ohne das weitere Hilfsmittel, wie beispielsweise eine Verpackung, notwendig sind. Die Konsistenz kann hierbei Gel- artig bis komplett fest sein.

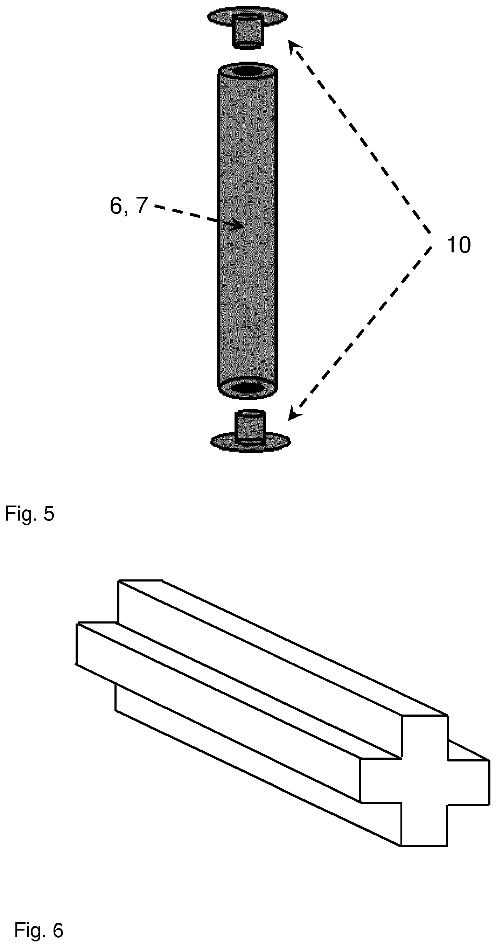

Die Zusammensetzung kann jegliche Form aufweisen. Mögliche Formen sind beispielsweise, jedoch nicht begrenzt auf, Stücke, Riegel, Sticks, Stifte, verschiedene geometrische Formen, wie Dreiecke, Kugeln, Kreuze und andere. In einer bevorzugten Ausführungsform handelt es sich um einen Stick. In einer besonders bevorzugten Ausführungsform ist der Stick derart geformt, dass er in eine entsprechende Öffnung in einer erfindungsgemäßen Vorrichtung, die als Kartusche, Applikator und/oder Duschbrause appliziert werden kann. Der dreidimensionale Stick hat vorzugsweise eine Grundfläche eines gleichschenkligen Kreuzes, wie beispielsweise im Beispiel 1 und Fig. 6dargestellt. Der Vorteil einer solchen Ausführungsform ist, dass Wasser alle Bereiche dieses gleichmäßig umfließen kann und in jedem Bereich ungefähr die gleiche Menge bzw. Fläche des Mittel zur Verfügung steht. Die erfindungsgemäße Zusammensetzung hat daher den Vorteil, dass diese im Wasserstrom leicht lösbar ist. In einer Ausführungsform weist der Stick die gleiche Grundform auf wie die Einfüllöffnung im Einfüllsystem 4.

Die Formgebung der Zusammensetzung kann durch Pressen (Extrusion) und/oder Gießen erreicht werden. Nachteilig an Sticks die mittels Extrusion hergestellt wurden, d.h. welche gepresst wurden, ist, dass diese im Vergleich zu den gegossenen Sticks relativ viele Sollbruchstellen aufweisen. Die Sollbruchstellen führen zu einem schnelleren Auflösen der Sticks, einer höheren Konzentration der Stoffe und/oder einer kürzeren Verwendungsdauer der Sticks. In einer bevorzugten Ausführungsform sind die Sticks daher mittels eines Gießverfahrens hergestellt. Die gegossenen Sticks weisen im Vergleich eine deutlich bessere Homogenisierung auf, wodurch das Dusch- bzw. Waschwasser eine bessere Anreicherung und Verteilung der im Stick enthaltenen Wirkstoffe aufweist.

Die Zusammensetzung zur Behandlung der Haut und/oder der Haare umfasst jegliche Behandlung. Die Behandlung kann beispielsweise die Pflege, Reinigung, Hygiene, Desinfektion, medizinische und/oder kosmetische Behandlung umfassen. Die Behandlung ist jedoch nicht beschränkt auf die vorherige Auflistung sondern kann jede im Stand der Technik bekannte Maßnahme umfassen.

Die erfindungsgemäßen Zubereitungen können, je nach Art der Formulierung, als weitere Hilfs- und Zusatzstoffe milde Tenside, Ölkörper, Emulgatoren, Konsistenzgeber, Verdickungsmittel, Polymere, Silikonverbindungen, Fette, Wachse, Stabilisatoren, biogene Wirkstoffe, Filmbildner, Quellmittel, Hydrotrope, Insektenrepellentien und/oder Solubilisatoren enthalten.

Die Zusammensetzung umfasst mindestens eine Fettphase die mindestens ein Tensid umfasst, welches zur Reinigung- und oder Aufschäumung dient. Tenside können nichtionische, anionische, kationische und/oder amphotere Tenside sein. Mögliche Tenside sind u.a. Saponine, Fettalkoholpolyglycolether, Fettalkoholsulfate, sulfierte Fettalkoholpolyglycolether Methylestersulfonate, Alkylglycoside, Alkylpolyglucoside, Phospholipide und/oder natürliche sowie synthetische Tenside.

Als milde Tenside werden insbesondere Tenside bezeichnet, die besonders naturverträglich sind. Beispiele für milde Tenside sind Fettalkoholpolyglycolethersulfate, Alkylethercarboxylate, alpha-Olefinsulfonate, Alkylbenzenesulfonate, Sulfosuccinate, Alkylamphoacetate, Betaine, Sultaine, Acylisothionate, Acylmethylisethionate, Taurate, Sarcosinate, APG's, Alkoholethoxylate, Sulfoacetate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine und/oder Proteinfettsäurekondensate. Bei den Proteinfettsäurekondensaten handelt es sich vorzugsweise um Proteinfettsäurekondensate auf Basis von Weizenproteinen.

In Ausführungsformen der vorliegenden Erfindung handelt es sich bei den Tensiden um 2-[3-(dodecanoylamino)propyl-dimethylazaniumyl]acetat (Cocamidopropyl Betaine, CAPB), (2R,3R,4S,5S,6R)-2-Decoxy-6-(hydroxymethyl)tetrahydropyran-3,4,5-triol (Decyl-Glucosid), 1,2-Propandiol (Propylenglycol), Kokosglucoside (Cocoglucoside) und Natriumcocoylisethionat. Die Fettphase weist in Abhängigkeit von der jeweiligen Anwendung Mengenanteile von 40 Gew.% bis 90Gew. % bezogen auf das Gesamtgewicht der Zusammensetzung auf. In einer bevorzugten Ausführungsform liegt der Fettphasenanteil bei 50 Gew.% bis 80 Gew. % bezogen auf das Gesamtgewicht. In einer besonders bevorzugten Ausführungsform liegt der Fettphasenanteil bei 60 Gew.% bis 70 Gew. % bezogen auf das Gesamtgewicht der Zusammensetzung. In der Fettphase können neben den tensidischen Bestandteilen, auch Öle, Wachse, Alkohole und/oder Fette verschiedener Herkunft enthalten sein. Diese sind beispielsweise pflanzliche, tierische, mineralische oder synthetische Fette und/oder Gemische hieraus. Beispiele solcher sind Panthenol, Sonnenblumenöl, Zitronenöl und/oder Limonenöl.

Als Ölkörper der vorliegenden Erfindung sind beispielsweise Guerbetalkohole auf Basis von Fettalkoholen mit 6 bis 32 18 Kohlenstoffatomen (C-Atomen), vorzugsweise 8 bis 18 10 Kohlenstoffatomen, Ester von linearen C6-C22-Fettsäuren mit linearen C5-C22-Fettalkoholen, Ester von verzweigten C5-C13-Carbonsäuren mit linearen C6-C22-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C5-C22-C32-Fettsäuren mit verzweigten Alkoholen, insbesondere 2-Ethylhexanol, Ester von Hydroxycarbonsäuren mit linearen oder verzweigten C6-C22-Fettalkoholen, insbesondere Dioctyl Malate; Ester von linearen und/oder verzweigten Fettsäuren mit mehrwertigen Alkoholen, wie z.B. Propylenglycol, Dimerdiol oder Trimertriol und/oder Guerbetalkoholen; Triglyceride auf Basis C5-C10-Fettsäuren; flüssige Mono-/Di-/ Triglyceridmischungen auf Basis von C5-C13-Fettsäuren; Ester von C5-C22-Fettalko- holen und/oder Guerbetalkoholen mit aromatischen Carbonsäuren, insbesondere Benzoesäure, Ester von C2-C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen; pflanzliche Öle; verzweigte primäre Alkohole; substituierte Cyclohexane; lineare und verzweigte C6-C22-Fettalko- holcarbonate; Guerbetcarbonate, Ester der Benzoesäure mit linearen und/oder verzweigten C5-C22-Alkoholen; lineare oder verzweigte, symmetrische oder unsymmetrische Dialkylether mit 6 bis 22 Kohlenstoffatomen pro Alkylgruppe; Ringöffnungsprodukte von epoxidierten Fettsäureestern mit Polyolen; Siliconöle und/oder aliphatische bzw. naphthenische Kohlenwasserstoffe, wie z. B. Squalan, Squalen und/oder Dialkylcyclohexane.

Als Emulgatoren kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
(2) C12/18-Fettsäuremonoerster und Fettsäuremonodiester von Anlagerungsprodukten von 1 bis 30 Mol Ethylenoxid an Glycerin;
(3) Glycerinmonoester und Glycerinmonoesterdiester und Sorbitanmonoester und Sorbitanmonodiester von gesättigten und ungesättigten Fettsäuren mit 6 bis 22 Kohlenstoffatomen und deren Ethylenoxidanlagerungsprodukte;
(4) Alkylglycoside und/oder Alkenylmonoglycoside und Alkenylmonooligoglycoside mit 8 bis 22 Kohlenstoffatomen im Alk(en)ylrest und deren ethoxylierte Analoga;
(5) Anlagerungsprodukte von 15 bis 60 Mol Ethylenoxid an Ricinusöl und/oder gehärtetes Ricinusöl;
(6) Polyolester und insbesondere Polyglycerinester;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C6/22-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG- alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß DE 1165574 PS und/oder Mischester von Fettsäuren mit 6 bis 22 Kohlenstoffatomen, Me-thylglucose und Polyolen, vorzugsweise Glycerin oder Polyglycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmonoester und Glycerindiester sowie Sorbitanmonoester und Sorbitandiester von Fettsäuren oder an Ricinusöl stellen bekannte, im Handel erhältliche Produkte dar.

Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C12/18- Fettsäuremonoester und Fettsäurediester von Anlagerungsprodukten von Ethylenoxid an Glycerin sind aus DE 2024051 PS als Rückfettungsmittel für kosmetische Zubereitungen bekannt.

Alkyl- und/oder Alkenylmono- und Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Typische Beispiele für geeignete Polyglycerinester sind Polyglyceryl-2 Dipolyhydroxystearate, Polyglycerin-3- Diisostearate, Polyglyceryl-4 Isostearate, Polyglyceryl-3 Oleate, Diisostearoyl Polyglyceryl-3 Diisostearate, Polyglyceryl-3 Me-thylglucose, Polyglyceryl-4 Caprate, Polyglyceryl-3 Cetyl Ether, Tego Gare APD 18, Polyglyceryl-3 Distearate und Polyglyceryl Polyricinoleate, Polyglyceryl Dimerate Isostearate sowie deren Gemische.

Als weitere Zusatzstoffe können Konsistenzgeber umfasst sein, bei denen es sich beispielsweise um Fettalkohole, Hydroxyfettalkohole mit 12 bis 22 und vorzugsweise 16 bis 18 Kohlenstoffatomen und daneben Partialglyceride, Fettsäuren, Guerbetalkohole mit einer Kettenlänge von C22-C32 oder Hydroxyfettsäuren handelt. Hydroxyfettalkohole mit 12 bis 22 Kohlenstoffatomen, insbesondere 16 bis 18 Kohlenstoffatomen sowie Guerbetalkohole mit einer Kettenlänge von 22-32 Kohlenstoffatomen haben sich als besonders geeignete Konsistenzgeber gezeigt. In einer besonders bevorzugten Ausführungsform weist die Zusammensetzung eine Kombination der Konsistenzgeber mit Alkyloligoglucosiden, Fettsäure-N- methylglucamiden gleicher Kettenlänge und/oder Polyglycerinpoly-12- hydroxystearaten auf.

Neben den zuvor genannten Konsistenzgebern können auch oder zusätzlich Fette und/ oder Wachse als Konsistenzgeber verwendet werden, um eine schöne Stifttextur zu erhalten.

Das in der Zusammensetzung verwendete Fett kann jedes im Stand der Technik bekannte Fett sein. Typische Beispiele für Fette sind Glyceride, und/oder Wachse. Als Wachse kommen u.a. natürliche Wachse, wie Beispielsweise Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse, chemisch modifizierte Wachse (Hartwachse), wie beispielsweise Montanesterwachse, Sasolwachse, hydrierte Jojobawachse sowie synthetische Wachse, wie z.B. Polyalkylenwachse und Polyethylenglycolwachse in Frage. Weitere Wachse, die für die Verwendung in der Zusammensetzung geeignet sind, stellen beispielsweise Wachse dar, die nach dem bekannten Fischer-Tropsch-Verfahren hergestellt wurden.

Die Zusammensetzung kann in einer weiteren Ausführungsform auch Stabilisatoren aufweisen. Diese sind beispielsweise Metallsalze von Fettsäuren, wie z.B. Magnesiumstearat, Zinkstearat und/oder Zinkricinoleat.

Geeignete Verdickungsmittel (Emulgatoren) für die Verwendung in der Zusammensetzung sind beispielsweise Aerosil-Typen (hydrophile Kieselsäuren), Polysaccharide, Xanthan-Gum, Acacia Senegal Gum, Caesalpinia spinosa gum, Gellan Gum, Sclerotium gum, Guar-Guar, Agar-Agar, Alginate, Tylosen, Carboxyme- thylcellulose, Hydroxyethylcellulose, höhermolekulare Polyethylenglycolmonoerster, und/oder Polyethylenglycolmonodiester von Fettsäuren, Polyacrylate, Polyvinylalkohol und/oder Polyvinylpyrrolidon.

In einer Ausführungsform der vorliegenden Erfindung kann die Zusammensetzung Polymere enthalten. Geeignete kationische Polymere sind beispielsweise kationische Cellulosederivate, wie z.B. quaternierte Hydroxyethylcellulose, kationische Stärke, Copolymere von Diallylammoniumsalzen und Acrylamiden, quaternierte Vinylpyrrolidon/Vinylimidazol-Polymere. Kondensationsprodukte von Polyglycolen und Aminen, quaternierte Kollagenpolypeptide, wie beispielsweise Lauryldimonium Hydroxypropyl hydrolysiertes Kollagen, quaternierte Weizenpolypeptide, Polyethylenimin, kationische Siliconpolymere, wie z.B. Amidomethicone, Copolymere der Adipinsäure und Dimethylaminohydroxypropyldiethylentriamin, Copolymere der Acrylsäure mit Dimethyldiallylammoniumchlorid, Polyaminopolyamide, wie beispielsweise in der FR 2252840 A beschrieben sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum und/oder quaternierte Ammoniumsalz-Polymere. Anionische, zwitterionische, amphotere oder nichtionische Polymere für die erfindungsgemäße Zusammensetzung sind beispielsweise Vinylacetat/Crotonsäure-Copolymere, Vinylpyrrolidon/Vinylacrylat-Copolymere, Vinylacetat/Butylmaleat/Isobornylacrylat- Copolymere, Methylvinylether/Maleinsäureanhydrid-Copolymere und deren Ester, unvernetzte undmit Polyolen vernetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxyproylmethacrylat-Copolymere, Polyvinylpyrrolidon, Vinylpyrrolidon/Vinylacetat- Copolymere, Vinylpyrrolidon/Dimethylaminoethylmethacrylat/Vinylcaprolactam- Terpolymere und/oder derivatisierte Celluloseether und Silicone.

Geeignete Siliconverbindungen für eine Ausfürungsform der Zusammensetzung sind beispielsweise Dimethylpolysiloxane, Methylphenylpolysiloxane, zyklische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können.

Neben den reinigenden und/oder pflegenden Eigenschaften kann die Zusammensetzung weitere Mittel enthalten, die zur Desinfizierung der Haut und/oder der Haare geeignet sind oder die der medizinischen Behandlung dienen können. Mittel zur Desinfektion der Haut und/oder der Haare können Polihexanid, Octenidin, jodenthaltende Mittel, wie beispielsweise Povidon-Jod und/oder Alkohole wie Ethanol, Isopropanol und/oder Phenoxyethanol sein. Sie sind jedoch nicht beschränkt auf die vorherigen Ausführungen, sondern können jedes bekannte Mittel im Stand der Technik sein. Weitere darin enthaltene Substanzen können beispielsweise sein die virginischen Zaubernuss (Hamamelis), Ringelblume (Calendula), Zink, Menthol, Antihistaminika, Antibiotika, Antimykotika, Hydrocortison, Aloe Vera, Honig, Kamille, Kurkuma, Heparin, Lavendelöl, Teebaumöl, Essigsäure, Salicylsäure, Harnstoff, Milchsäure, Dithranol, Calcipotriol, Tacalcitol, Calcitriol, Retinoiden, verschiedene Salze, Nachtkerzenöl, Ciclosporin, Azathioprin, Calcineurin, Tacrolimus (Protopic), Pimecrolimus, heilpflanzliche Extrakte, wie beispielsweise Salbei, Lavendel, Johanniskraut, Pfefferminze, Thymian und/oder Chili, oder Kombinationen aus diesen.

Weitere biogenen Wirkstoffe sind beispielsweise Tocopherol, Tocopherolacetat, Tocopherolpalmitat, Ascorbinsäure und ihre Ester, insbesondere Ascorbylisostearat, Desoxyribonucleinsäure, Retinol, Bisabolol, Allantoin, Phytantriol, Panthenol, AHA- Säuren, Aminosäuren, Hyaluronsäure von 10 Kilodalton bis 1,8 millionen Kilodalton, Ceramide, Pseudoceramide, essentielle Öle und Vitaminkomplexe. Die Substanzen sind jedoch nicht beschränkt auf die vorherige Auflistung sondern können jede Substanz zur Milderung, Hemmung und/oder Bekämpfung von Krankheiten umfassen.

Die Parfüm- und/oder Farbphase kann verschiedene Stoffe enthalten, die bestimmte Düfte oder Farben beim Baden, Duschen und/oder Waschen freigibt und/oder entfaltet. Duftstoffe können in Form von Ölen oder Parfüm hierbei eingebracht werden. In einer Ausführungsform der vorliegenden Erfindung weist die Zusammensetzung eine Kombination von Eukalyptusöl, Pfefferminzöl und Latschenkiefer auf, welche insbesondere bei Erkältungen zum besseren Wohlbefinden beiträgt. Duftstoffe können jedoch jeder bekannte Duft oder eine Kombination aus Düften sein, die jedoch vorzugsweise aus natürlich vorkommenden Stoffen gewonnen werden.

Duftstoffe, die der Zusammensetzung zugesetzt werden können, können eine oder mehrere, bevorzugt ein oder zwei Hydroxylgruppen verfügen, welche veresterbar oder verestert sind, unabhängig davon, wie das Molekül weiter aufgebaut ist. So lassen sich auch Salicylsäureester als Duftstoffalkohole einsetzen. Geeignet sind dabei alle Kombinationen von geometrischen Isomeren. Ebenfalls ist es möglich, Ester dieser Verbindungen einzusetzen, beispielsweise Anethol (1-Methoxy-4-(1- propenylbenzol) oder Linalylacetat. In einer besonders bevorzugten Ausführungsform werden Duftstoffalkohole mit ein oder zwei freien Hydroxylgruppen eingesetzt. Aus der großen Gruppe der Duftstoffalkohole, zu denen auch die Terpenalkohole gehören, lassen sich bevorzugte Vertreter mit mindestens einer freien Hydroxylgruppe nennen, so dass im Rahmen der vorliegenden Erfindung bevorzugt sind: 1O-Undecen-1-ol,2,6-Dimethylheptan-2-ol,2-Methylbutanol, 2- Methylpentanol, 2-Phenylpropanol, 2-tert-Butycyclohexanol, 3,5,5-Trimethylcyclohexanol, 3-Hexanol, 3-Methyl-5-phenylpentanol, 3-Octanol,3- Phenylpropanol, 4-Heptenol, 4-Isopropylcyclohexanol, 4-tert-Butycyclohexanol, 6,8-Dimethyl-2-nonanol, 6-Nonen-1-ol, 9-Decen-1-ol, alpha-Methylbenzylalkohol, 1-Hydroxy-4-(1-propenylbenzol), Amylsalicylat, Benzylalkohol, Benzylsalicylat, Butylsalicylat, Citronellole, Cyclohexylsalicylat, Decanol, Dihydromyrcenol, Dimethylbenzylcarbinol, Dimethylheptanol, Dimethyloctanol, Ethylsalicylat, Ethylvanilin, Eugenol, Heptanol, Hexylsalicylat, Isoeugenol, Isopulegol, Menthol, Myrtenol, n-Hexanol, Nerol, Nonanol, Octanol, para-Menthan-7-ol, Phenylethylalkohol, Phenylsalicylat, Tetrahydrogeraniol, Tetrahydrolinalool, Thymol, trans-2-cis-6-Nonadicnol, trans-2-Nonen-1-ol, trans-2- Octenol, Undecanol, Zimtalkohol. Bevorzugt sind beispielsweise Eugenol und dessen Derivate, 1-Hydroxy-4-(1-propenylbenzol), Isoeugenol, Citronellole und/oder Menthol. Unter Terpenen sind erfindungsgemäß alle aus Isopreneinheiten aufgebauten Naturstoffe und Derivate zu verstehen, wobei beispielsweise Farnesol, Patchoulialkohol, Squalen, Geraniol bevorzugt sind. Gemäß einer besonders bevorzugten Ausführungsform sind unter Terpenen insbesondere die Mono-, Sesqui- und/oder Diterpene zu verstehen. Es können azyklische, monozyklische und/oder bizyklische sowie höherzyklische Mono-, Sesqui- und/oder Diterpene eingesetzt werden. Unter den Derivaten von Monoterpenen, Sesquiterpenen bzw. Diterpenen sind beispielsweise Alkohole, wie zum Beispiel Farnesol und deren Ether, Säuren, wie zum Beispiel Famesolsäure, sowie deren Ester und andere funktionelle Gruppen tragende Mono-, Sesqui- bzw. Diterpene zu verstehen. Geeignet sind dabei alle Kombinationen von geometrischen Isomeren. Ebenfalls darunter fällt a-Famesen (3,7,11-Trimethyl-1 ,3,6, 10-Dodekatetraen) sowie ß-Famesen (7,11-Dimethyl-3- Methylen-1 ,6,10-Dodekatrien) und Nerolidol (3,7,11-Trimethyl-1 ,6,10-Dodekatrien- 3-ol) sowie Patchoulialkohol, Bisabolen, Sesquiphellandren, Zingiberen, Cadinen, Caryophyllene (insbesondere a-Caryophyllen (Humulen) und β-Caryophyllen), aryl-Tumeron, Tumeron, Xanthorrhizol, Vulgären und ß-Selinen. Als Monoterpene sind beispielsweise a- bzw. ß-Ocimen, Linalool, Linalylacetat, Borneole, Isoborneole, Carene, Terpineole, p-Menthadiene, Limonen, Nerol, Nerolsäure, Geraniol, Geraniumsäure, a- bzw. ß-Phellandren und/oder Thujon, insbesondere Geraniol, Linalool und/oder Thujon bevorzugt geeignet. Beispiele für die Diterpene sei hier Geranylgeraniol (3,7,11,15-Tetramethyl-2,6, 10,14-Hexadekatet-raen-1-ol) sowie seine Isomere und Derivate. Es können ebenfalls Pflanzenextrakte eingesetzt werden, die Duftstoffalkohole und/oder Terpene, insbesondere Mono-, Sesqui- und/oder Diterpene, enthalten, beispielsweise Geraniumöl, Rosenöl, Patchouliöl, Fichtennadelöl, Orangenöl, Orangenblütenöl, Lavendelöl, Limettenöl, Jasminöl, Pfefferminzöl, Basilikumöl, Citronellöl, Zypressenöl, Zedernblätteröl, Zedernholzöl, Korianderöl, Rosenholzöl, Thymianöl, Pimentöl, Ingweröl oder Nelkenöl, insbesondere Patchouliöl, Nelkenöl, Zypressenöl, Zedernholzöl und/oder Anisöl.

Neben der Parfümphase kann die Zusammensetzung zusätzlich oder alternativ in einer Ausführungsform auch Mittel zur Färbung umfassen. Diese Mittel können insbesondere bei Kindern dazu beitragen, dass diese das Duschen als Spaß wahrnehmen und so diese zum Duschen, Baden und/oder Waschen animieren. Die Zusammensetzung können zum Spaßbaden auch Glitzerteile und/oder Glitterteile enthalten, die jedoch vorzugsweise eine geringe Größe von wenigen Millimetern aufweisen, um Verstopfungen zu vermeiden. In einer bevorzugten Ausführungsform sind die Glitzerteile und/oder Glitterteile wasserlöslich, sodass sie sich nach einer bestimmten Zeit im Wasser selber auflösen.

In einer Ausführungsform der vorliegenden Erfindung kann die Zusammensetzung einen Wasseranteil aufweisen. Der Wasseranteil kann dazu geeignet sein bestimmte Stoffe oder Bestandteile, die in polaren Lösungsmitteln mischbar sind, aufzunehmen und/oder die Konsistenz der kompletten Zusammensetzung zu definieren. In einer bevorzugten Ausführungsform ist die Zusammensetzung jedoch ganz oder teilweise dehydratisiert. Der Vorteil der dehydratisierten Form ist, dass auf Mittel zur Konservierung ganz oder teilweise verzichtet werden kann, wodurch eine bessere Verträglichkeit der Zusammensetzung für Haut und/oder Haar gegeben ist. Zusätzlich führt die Dehydratisierung zu einer höheren Langlebigkeit und Haltbarkeit der Zusammensetzungen.

Die vorliegende Erfindung betrifft auch ein Verfahren zur Anreicherung von Wasser mit Medium 7 mit einer der oben aufgezeigten und in den Ansprüchen 1-7 definierten Zusammensetzung zum Reinigen, Pflegen und/oder zur Behandlung. Bei dem Verfahren kann das Wasser mit unterschiedlichen Medien 7 mit einer der oben genannten Zusammensetzungen zum Reinigen, Pflegen und/oder zur Behandlung der Haut und/oder Haaren gleichzeitig oder separat angereichert werden.

Im Verfahren wird das Wasser aus einer Leitung über ein Verteiler 3 in mindestens eine der mindestens zwei getrennten Anreicherungskammern 2 geleitet. Diese können mindestens ein Medium 7 zur Reinigung, Pflege und/oder Behandlung der Haut und/oder Haaren enthalten. Die Ausgestaltung der Anreicherungskammer 2 ist hierbei, wie in der oben beschriebenen Vorrichtung.

Das durch die Anreicherungskammern 2 durchgeführte Wasser aus der Leitung umspült und/oder durchspült das Medium 7 und wird mit dem Medium 7 vermischt.

Im weiteren Verlauf fließt das angereicherte Wasser durch eine Misch- und/oder Verwirbelungskammer 5, die zu einer Vermischung des Materials 7 und des Wassers führt. In einer Ausführungsform erfolgt die Vermischung durch eine regelbare Sauerstoffdüse 11. Die Menge an zugeführtem Sauerstoff ermöglicht eine individuelle Regulierung der Schaumproduktion als auch der Wasserstrahlhärte. Bevorzugte Ausgestaltungen der Düse hierbei in der oben dargestellten Vorrichtung 1 beschrieben.

Für nähere Ausführungen zu den in dem Verfahren verwendeten Bauteilen und Elementen wird auf die Ausführungen zur Vorrichtung 1 zuvor verwiesen sowie des verwendeten Mediums 7 mit der zuvor beschriebenen Zusammensetzung.

Diese und andere Ausführungsformen der vorliegenden Erfindung werden in der Beschreibung und den Beispielen offenbart und sind durch diese umfasst. Weitere Literatur über eine bekannte der Materialien, Verfahren und Anwendungen die in Übereinstimmung mit der vorliegenden Erfindung verwendet werden können, können aus öffentlichen Bibliotheken und Datenbanken, beispielsweise unter Verwendung elektronischer Geräte aufgerufen werden. Ein vollständigeres Verständnis der Erfindung kann durch Bezugnahme auf die Figuren und Beispiele erhalten werden, die zum Zweck der Illustration bereitgestellt wurden und den Umfang der Erfindung nicht beschränken sollen.

### Beispiele

### Beispiel 1: Duschzusammensetzung mit verschiedenen Wirkstoffen

Die erfindungsgemäße Zusammensetzung die vorzugsweise die Form eines dreidimensionalen Sticks, der eine Grundfläche eines gleichschenkligen Kreuzes, aufweist, kann zu unterschiedlichen Anlässen oder Stimmungslagen verwendet werden. Der Stick wird vorzugsweise in einer zugehörigen Vorrichtung eingesetzt oder ausgetauscht. In den Tab. 1 bis 5 sind Inhaltsstoffe möglicher erfindungsgemäßer Zusammensetzungen dargestellt, welche vorzugsweise als Sticks zum Einsatz kommen.

**Tab. 1 Duschzusammensetzung für Kinder**

| **Phase** | **Name** | **Menge [%]** |
|---|---|---|
| *Fettphase* | Cocamidopropyl-Betain | 10 |
| | Decyl-Glucosid | 15 |
| | Propylenglycol | 28 |
| | Coco-Glucosid | 5 |
| | Natrium Cocoyl-Isethionat | 10 |
| *Verdickerphase* | Natriumstearat | 30 |
| *Parfümphase* | Vanille und Schokolade | 2 |

**Tab. 2 Duschzusammensetzung Wellness**

| **Phase** | **Name** | **Menge [%]** |
|---|---|---|
| *Fettphase* | Cocamidopropyl-Betain | 10 |
| | Decyl-Glucosid | 15 |
| | Propylenglycol | 28 |
| | Coco-Glucosid | 5 |
| | Natrium Cocoyl-Isethionat | 10 |
| *Verdickerphase* | Natriumstearat | 30 |
| *Parfümphase* | Wellness PCMF Öko der | 2 |

### Firma Düllberg

**Tab. 3 Duschzusammensetzung für die Aktivierung der Haut**

| **Phase** | **Name** | **Menge [%]** |
|---|---|---|
| *Fettphase* | Cocamidopropyl Betain | 8 |
| | Decyl-Glucoside | 13 |
| | Propylenglycol | 13 |
| | Coco-Glucoside | 5 |
| | Natrium Cocoyl-Isethionat | 8 |
| | Zinc PCA | 1 |
| | Niacinamide | 5 |
| | Harnstoff (Urea) | 10 |
| | Panthenol, Wasser, Zitronensäure | 5 |
| *Verdickerphase* | Natriumstearat | 30 |
| *Parfümphase* | Nice Day 44411 der Firma Luzi AG | 2 |

**Tab. 4 Duschzusammensetzung zum Wachwerden**

| **Phase** | **Name** | **Menge** |
|---|---|---|
| *Fettphase* | Cocamidopropyl Betain | 10 |
| | Decyl-Glucoside | 15 |
| | Propylenglycol | 25 |
| | Coco-Glucoside | 5 |
| | Natrium Cocoyl-Isethionat | 10 |
| | Helianthus Annuus (Sonnenblumen) Samenöl, Paullinia Cupana | 3 |
| | Samenextrakt Extrakt | |
| *Verdickerphase* | Natriumstearat | 30 |
| *Parfümphase* | Citrus Aurantium Dulcis (Orangenschalenöl) | 2 |

**Tab. 5 Duschzusammensetzung für Erkältungen**

| **Phase** | **Name** | **Menge [%]** |
|---|---|---|
| *Fettphase* | Cocamidopropyl Betain | 10 |
| | Decyl-Glucoside | 15 |
| | Propylenglycol | 24 |
| | Coco-Glucosid | 5 |
| | Natrium Cocoyl-Isethionat | 10 |
| | Helianthus Annuus (Sonnenblumen) Samenöl, Paullinia Cupana | 3 |
| | Samenextrakt Extrakt | |
| *Verdickerphase* | Natriumstearat | 30 |
| *Parfümphase* | Eucalyptus Globulus Blattöl | 2 |
| | Mentha Piperita (Pfefferminzöl) | 2 |
| | Pinus Mugo Blattöl (Latschenkiefer), Limone | 2 |

Wie den Tab. 1 bis 5 zu entnehmen ist, weisen die Duschzusammensetzungen drei Hauptphasen auf, nämlich die Fettphase, die Verdickerphase und die Parfümphase. Die Fettphase besteht bei allen Zusammensetzungen insbesondere aus den Bestandteilen Cocamidopropyl Betain, Decyl-Glucosid, Propylenglycol, Coco- Glucosid und Natrium Cocoyl Isethionat. Die Mengen der Inhaltsstoffe der Fettphase unterscheiden sich nur gering. Cocamidopropyl Betain macht 8 bis 10 Gew.% der Geamtzusammensetzung aus, das Decyl-Glucosid 13 bis 15 Gew.%, das Propylenglycol 13 bis 28 Gew.%, das Coco-Glucosid 5 Gew.% und das Natrium Cocoyl-Isethionat 8 bis 10 Gew.% aus. Weitere Bestandteile können in der Fettphase enthalten sein, die beispielsweise eine hautpflegende Wirkung aufzeigen, wie Harnstoff, in Tab. 3. Natriumstearat, das wie Kaliumstearat in vielen Seifen Verwendung finden, wird in den Zusammensetzungen vorzugsweise als Verdickungsmittel bzw. Emulgator verwendet. Vorzugsweise macht der Verdicker 30 Gew.% der Gesamtzusammensetzung aus. Die Parfümphase kann neben verschiedensten Duftstoffen auch Öle oder andere duftende Substanzen enthalten, die einen bestimmten Geruch verströmen. Die Menge der Duftstoffe kann zwischen 0 und 10 Gew.% variieren, je nach gewünschter Intensität des Duftes.

### Beispiel 2: Vorrichtung zur Anreicherung von Wasser mit drehbarer Kammer

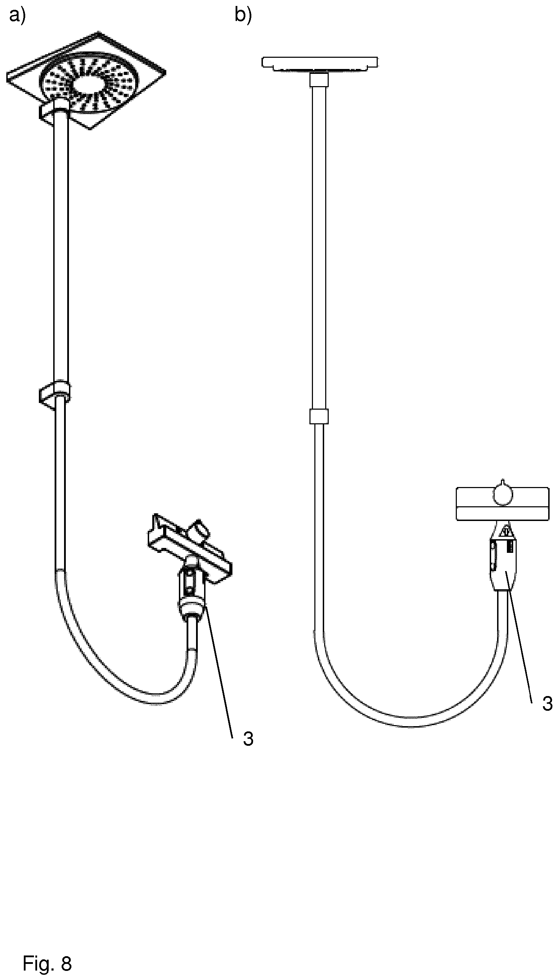

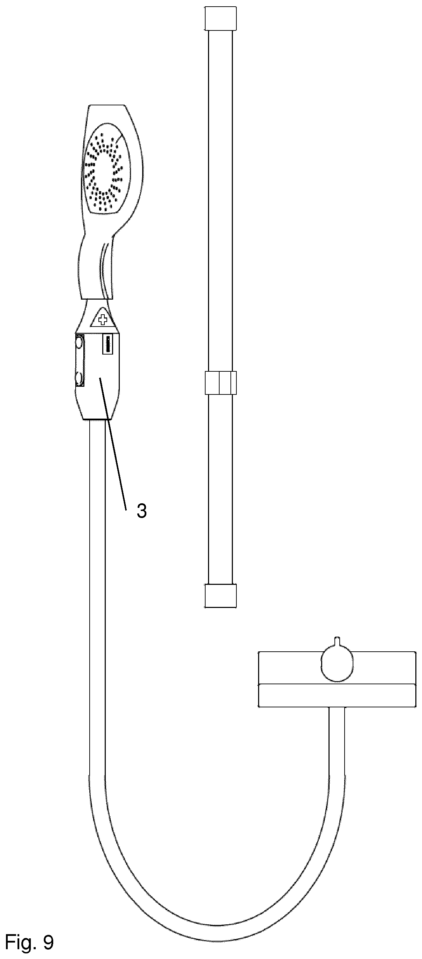

Die Vorrichtung zur Anreicherung von Wasser 1 kann in einer Ausführungsform, wie in Fig. 8 dargestellt, ein Element sein, welches zwischen eine herkömmliche Armatur, Duschvorrichtung oder ähnliche und den Wasserzufluss geschaltet werden kann, wie den Fig. 8 und 9 zu entnehmen ist. Die Verbindung erfolgt hierbei vorzugsweise über die standardisierten DIN-Verbindungen. Jedoch können auch Adapter zwischengeschaltet sein. Das Einfüllsystem 4 der erfindungsgemäßen Vorrichtung 1 zum Auswechseln und/oder Befüllen der Kammer 2 kann Scharniergelenke enthalten, die es ermöglichen die den Kammerzusammenschluss aus den mindestens zwei Kammern 2, komplett zu drehen und eine Befüllung der mindestens einen Kammer zu ermöglichen. Die Drehbewegung des Kammerzusammenschlusses kann verhindert werden, durch beispielsweise eine Klickverbindung. Die ineinandergreifenden Elemente rasten ein, wodurch eine ungewollte Bewegung verhindert wird. Die Klickverbindung kann auch durch entsprechende Schieber geschlossen werden.

### Beispiel 3: Vorrichtung zur Anreicherung von Wasser mit verschiebbarer Klappe

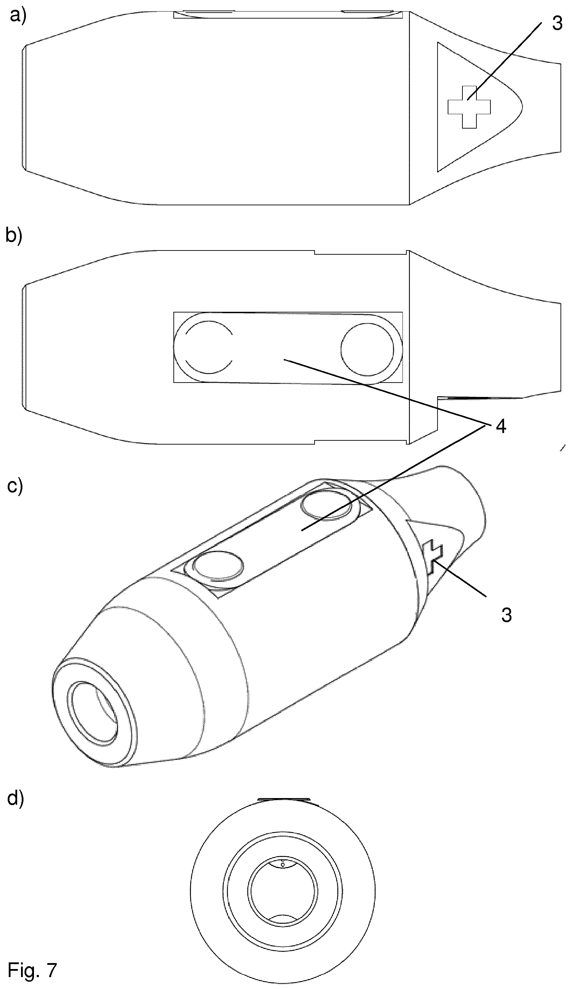

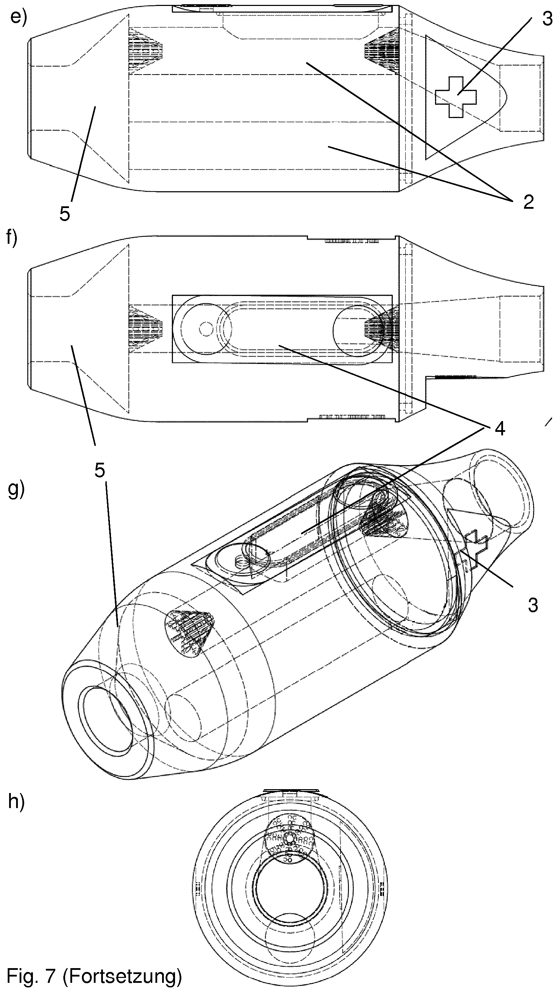

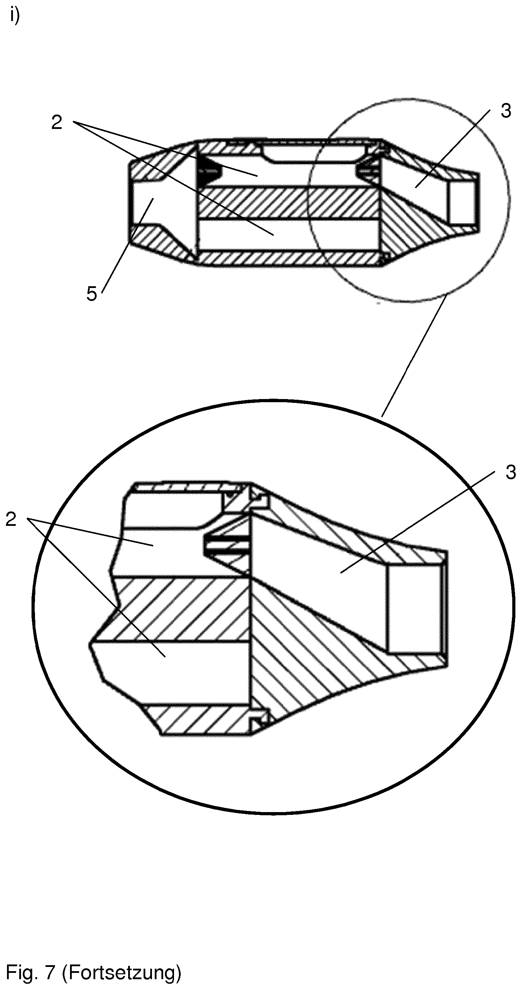

Die Vorrichtung zur Anreicherung von Wasser 1 kann in einer Ausführungsform, wie in Fig. 7 dargestellt, ein Element sein, welches zwischen einer bereits bestehenden Vorrichtung zur Abgabe von Wasser und dem Wasserschlauch bzw. an dieser befestigt ist, wie den Fig. 8 und 9 zu entnehmen ist. Die beispielhafte Ausführung der Vorrichtung weist zwei Kammern 2 auf, die sich auf den gegenüberliegenden Seiten innerhalb der Vorrichtung 1 befinden. Die mindestens eine Kammer 2 kann hierbei durch eine verschiebbares Einfüllsystem 4, nämlich eine Klappe geöffnet oder verschlossen werden. Das Einfüllsystem 4 besteht aus einer Klappe, die an einer Seite mit der Vorrichtung 1 bewegbar befestigt ist. Die bewegbare Befestigung ist vorzugsweise ein Scharnier oder ein Band. Die Klappe kann durch einen Schieber geöffnet oder verschlossen werden, indem der Schieber in einem bestimmten Bewegungsbereich bewegt wird. Ein Verteiler 3 erlaubt die Verstellung der des Wasserflusses in die Vorrichtung 1 und mindestens eine der beiden Kammern 2. Durch den Wasserdurchlauf durch eine der beiden Kammern 2, in denen sich dieses beispielsweise mit Medium 7 anreichern kann, fließt das Wasser in die Misch- bzw. Verwirbelungskammer 5, in der Wasser aus den beiden Kammern 2 vermischt wird, oder beim Wasserfluss durch einer Kammer, in der Medium enthalten ist, das Medium 7 zusätzlich mit dem Wasser vermischt wird und/oder mit zusätzlichem Sauerstoff durch eine Düse angereichert wird, um beispielsweise mehr Schaum zu generieren und/oder den Wasserverbrauch zu senken.

## Patentansprüche

1. Feste, wasserlösliche Zusammensetzung umfassend
a) eine Fettphase umfassend eine Kombination aus
- 8 bis 10% 2-[3-(dodecanoylamino)propyl-dimethylazaniumyl]acetat (Cocamidopropyl Betaine, CAPB);
- 13 bis 15 % (2R,3R,4S,5S,6R)-2-Decoxy-6-(hydroxymethyl)tetrahydropyran-3,4,5-triol (Decyl-G lucosid);
- 13 bis 28 % 1,2-Propandiol (Propylenglycol);
- 5 % Kokosglucoside (Cocoglucoside); und
- 8 bis 10 % Natriumcocoylisethionat.

2. Zusammensetzung nach Anspruch 1, wobei die Zusammensetzung zusätzlich Mittel zur Desinfizierung, medizinischen Behandlung, Pflege der Haut und/oder der Haare umfasst.

3. Zusammensetzung nach Anspruch 2, wobei die zusätzlichen Mittel Polihexanid, Octenidin, jodenthaltende Mittel, Alkohole, die virginischen Zaubernuss (Hamamelis), Ringelblume (Calendula), Zink, Menthol, Antihistaminika, Antibiotika, Antimykotika, Hydrocortison, Aloe Vera, Honig, Kamille, Kurkuma, Heparin, Lavendelöl, Teebaumöl, Essigsäure, Salicylsäure, Harnstoff, Milchsäure, Dithranol, Calcipotriol, Tacalcitol, Calcitriol, Retinoiden, verschiedene Salze, Nachtkerzenöl, Ciclosporin, Azathioprin, Calcineurin, Tacrolimus (Protopic), Pimecrolimus, heilpflanzliche Extrakte sind oder eine Kombinationen aus diesen.

4. Zusammensetzung nach Anspruch 1 oder 2, wobei die Zusammensetzung zusätzlich eine Parfüm- und/oder Farbphase umfasst.

5. Zusammensetzung nach einem der Ansprüche 1 bis 4, wobei die Zusammensetzung Glitzerteile und/oder Glitterteile umfasst.

6. Zusammensetzung nach einem der Ansprüche 1 bis 5, wobei diese die Form eines dreidimensionalen Sticks aufweist.

7. Zusammensetzung nach einem der Ansprüche 1 bis 6, wobei die Zusammensetzung die Form eines dreidimensionalen Sticks mit einer Grundfläche eines gleichschenkligen Kreuzes aufweist.

8. Verfahren zur Anreicherung von Wasser mit einer Zusammensetzung nach einem der Ansprüche 1 bis 7, umfassend folgende Schritte
a) Einfüllen der Zusammensetzung in eine Anreicherungskammer einer Vorrichtung mit mindestens zwei getrennten Anreicherungskammern (2);
b) Einleitung von Wasser aus der Leitung wird über einen Verteiler (3) der Vorrichtung in die mit der Zusammensetzung gefüllte Anreicherungskammer (2);
c) Umspülen und/oder Durchspülten der Zusammensetzung mit dem durchgeführten Wasser aus der Leitung;
d) Einleitung des angereicherten Wassers mit der Zusammensetzung in eine Misch- und/oder Verwirbelungskammer (5), die zu einer Vermischung der Zusammensetzung und des Wassers führt; und
e) Ausgabe des angereicherten Wassers mit der Zusammensetzung.

## Claims

1. Solid, water-soluble composition comprising
a) a fatty phase comprising a combination of
- 8 to 10% of 2-[3-(dodecanoylamino)propyl dimethylazaniumyl] acetate (cocamidopropyl betaine, CAPB);
- 13 to 15 % (2R,3R,4S,5S,6R)-2-decoxy-6-(hydroxymethyl)tetrahydropyran-3,4,5-triol (decyl glucoside);
- 13 to 28% 1,2-propanediol (propylene glycol);
- 5% coco-glucoside (coco-glucoside); and
- 8 to 10% sodium cocoyl isethionate.

2. Composition according to claim 1, wherein the composition comprises additional agents for disinfection, medical treatment, care of the skin and/or hair.

3. Composition according to claim 2, wherein the additional agents are polihexanide, octenidine, iodine-containing agents, alcohols, Virginian witch hazel (Hamamelis), marigold (Calendula), zinc, menthol, antihistamines, antibiotics, antimycotics, hydrocortisone, aloe vera, honey, chamomile, turmeric, heparin, lavender oil, tea tree oil, acetic acid, salicylic acid, urea, lactic acid, dithranol, calcipotriol, tacalcitol, calcitriol, retinoids, various salts, evening primrose oil, ciclosporin, azathioprin, calcineurin, tacrolimus (Protopic), pimecrolimus, medical plant extracts or a combination of these.

4. Composition according to claim 1 or 2, wherein the composition additionally comprises a perfume and/or color phase.

5. Composition according to one of claims 1 to 4, wherein the composition comprises twinkle particles and/or glitter particles.

6. Composition according to one of Claims 1 to 5, wherein it is in the form of a three-dimensional stick.

7. Composition according to any one of claims 1 to 6, wherein the composition is in the form of a three-dimensional stick having a base of an isosceles cross.

8. Method for enriching water with a composition according to any one of claims 1 to 7, comprising the steps of
a) introducing the composition into an enrichment chamber of a device having at least two separate enrichment chambers (2);
b) introducing tap water via a distributor (3) of the device into the enrichment chamber (2) filled with the composition;
c) washing around and/or flushing through the composition with the water from the pipe;
d) introducing the enriched water with the composition into a mixing and/or swirling chamber (5), which leads to the composition and the water being mixed; and
e) dispensing the enriched water with the composition.

## Revendications

1. Composition solide hydrosoluble comprenant
a) une phase grasse comprenant une combinaison de
- 8 à 10 % d'acétate de 2-[3-(dodécanoylamino)propyl-diméthylazaniumyl] (cocamidopropyl bétaïne, CAPB) ;
- 13 à 15 % de (2R,3R,4S,5S,6R)-2-décoxy-6-(hydroxyméthyl)tétrahydropyran-3,4,5-triol (décyl glucoside) ;
- 13 à 28 % de 1,2-propanediol (propylène glycol) ;
- 5 % de coco glucoside ; et
- 8 à 10 % de cocoyliséthionate de sodium.

2. Composition selon la revendication 1, dans laquelle la composition comprend en supplément des agents de désinfection, de traitement médical, de soin de la peau et/ou des cheveux.

3. Composition selon la revendication 2, dans laquelle les agents supplémentaires sont le polyhexanide, l'octénidine, les agents contenant de l'iode, les alcools, la noix vierge magique (hamamélis), le calendula, le zinc, le menthol, les antihistaminiques, les antibiotiques, les antifongiques, l'hydrocortisone, l'aloe vera, le miel, la camomille, le curcuma, l'héparine, l'huile de lavande, l'huile d'arbre à thé, l'acide acétique, l'acide salicylique, l'urée, l'acide lactique, le dithranol, le calcipotriol, le tacalcitol, le calcitriol, les rétinoïnes, les différents sels, l'huile d'onagre, la ciclosporine, l'azathioprine, la calcinéurine, le tacrolimus (Protopic), le pimécrolimus, les extraits de plantes médicinales ou une combinaison de ceux-ci.

4. Composition selon la revendication 1 ou 2, dans laquelle la composition comprend en supplément une phase parfumée et/ou colorée.

5. Composition selon l'une quelconque des revendications 1 à 4, dans laquelle la composition comprend des particules pailletées et/ou des particules scintillantes.

6. Composition selon l'une quelconque des revendications 1 à 5, dans laquelle celle-ci présente la forme d'un bâtonnet tridimensionnel.

7. Composition selon l'une quelconque des revendications 1 à 6, dans laquelle la composition présente la forme d'un bâtonnet tridimensionnel avec une surface de base d'une croix isocèle.

8. Procédé d'enrichissement de l'eau avec une composition selon l'une quelconque des revendications 1 à 7, comprenant les étapes suivantes
a) de remplissage de la composition dans une chambre d'enrichissement d'un dispositif comportant au moins deux chambres d'enrichissement séparées (2) ;
b) d'introduction d'eau provenant de la conduite par l'intermédiaire d'un distributeur (3) du dispositif dans la chambre d'enrichissement (2) remplie de la composition ;
c) de rinçage et/ou de rinçage complet de la composition avec l'eau acheminée provenant de la conduite ;
d) d'introduction de l'eau enrichie avec la composition dans une chambre de mélange et/ou de turbulence (5), ce qui donne lieu à un mélange de la composition et de l'eau ; et
e) de distribution de l'eau enrichie avec la composition.
